# EUROPÄISCHE PATENTSCHRIFT

(11) **EP 3 247 299 B1**
(45) Veröffentlichungstag und Bekanntmachung des Hinweises auf die Patenterteilung: **25.12.2019**
(21) Anmeldenummer: 16701587.4
(22) Anmeldetag: 21.01.2016
(51) Int. Cl.: A61B 34/30, A61B 34/00, A61B 90/50

(54) **VORRICHTUNG ZUM HALTEN UND BEWEGEN EINES LAPAROSKOPS WÄHREND EINER OPERATION**
DEVICE FOR RETAINING AND MOVING A LAPAROSCOPIC INSTRUMENT DURING AN OPERATION
DISPOSITIF POUR MAINTENIR ET METTRE EN MOUVEMENT UN LAPAROSCOPE PENDANT UNE OPÉRATION

(30) Priorität: 23.01.2015 DE 102015101018
(43) Veröffentlichungstag der Anmeldung: 29.11.2017
(73) Patentinhaber: Maquet GmbH, 76437 Rastatt (DE)
(72) Erfinder: BERNHART, Michael, 76275 Ettlingen (DE); GINZINGER, Lena, 72072 Tübingen (DE); OBERT, Mike, 76593 Gernsbach (DE)
(74) Vertreter: Zacco GmbH
(86) Internationale Anmeldenummer: PCT/EP2016/051190
(87) Internationale Veröffentlichungsnummer: WO 2016/116538

(56) Entgegenhaltungen:
- US-A- 4 551 058
- US-A- 5 078 140
- US-A1- 2003 167 061
- US-A1- 2003 221 504
- US-A1- 2005 234 293
- US-A1- 2007 089 557
- US-A1- 2012 041 263

## Beschreibung

Die Erfindung betrifft eine Vorrichtung zum Halten und Bewegen eines Laparoskops während einer Operation, die eine Befestigungseinheit zur Befestigung der Vorrichtung an einem Haltearm sowie eine Aufnahmeeinheit zum Halten des Laparoskops umfasst.

Während laparoskopischen Eingriffen betrachtet der Operateur den Operationssitus indirekt über einen Monitor, auf dem die laparoskopischen Instrumente zu sehen sind, mit denen er die anatomischen Strukturen und Organe der Patienten manipuliert. Das auf dem Monitor in Echtzeit gezeigte Bild wird mit einer Kamera aufgenommen, die auf einer ebenfalls laparoskopisch eingeführten Optik befestigt ist und auf den Operationssitus ausgerichtet ist.

Das Laparoskop mit der Kamera muss hierbei immer auf denjenigen Bereich gerichtet sein, in dem aktuell der Operateur arbeitet. Soll der zu betrachtende Bildausschnitt geändert werden, so muss das Laparoskop mit der Kamera bewegt werden, so dass der gewünschte Bereich wieder im Zentrum des dargestellten Bildes ist.

Alle bei der Nachführung notwendigen Bewegungen des Laparoskops lassen sich insbesondere aus drei Bewegungen zusammensetzen. Die erste Bewegung, die sogenannte PAN-Bewegung, stellt ein Schwenken des Laparoskops um den Trokarpunkt dar, d.h., dass eine Rotation um eine z-Achse senkrecht zur Patientenlagerfläche erfolgt. Die zweite notwendige Bewegung ist die sogenannte TILT-Bewegung, bei der ein Kippen des Laparoskops um den Trokarpunkt erfolgt. Somit erfolgt bei der TILT-Bewegung eine Rotation um eine Drehachse in der xy-Ebene, also in einer Ebene parallel zur Patientenlagerfläche. Die dritte notwendige Bewegung ist die ZOOM-Bewegung, bei der ein Vergrößern oder Verkleinern des sichtbaren Bildausschnittes durch Verschieben des Laparoskops entlang seiner Längsachse erfolgt. Bei dieser ZOOM-Bewegung wird das Laparoskop somit translatorisch entlang einer Achse durch den Trokarpunkt bewegt.

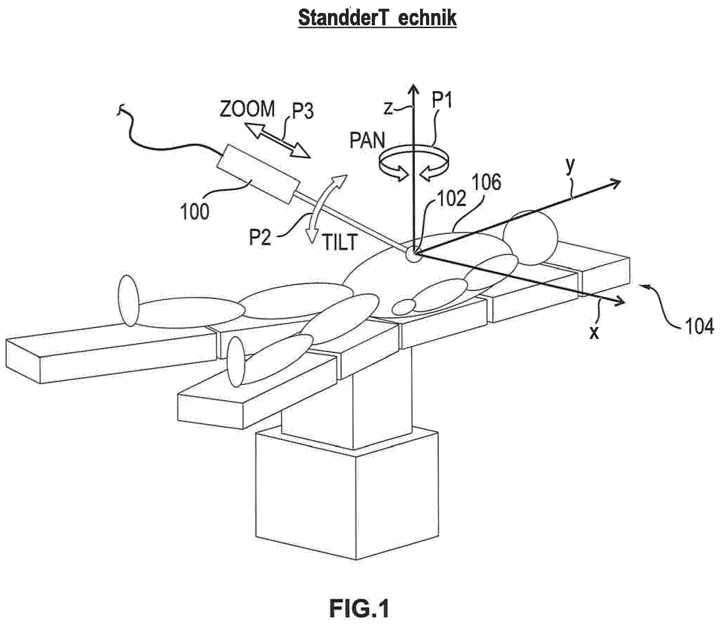

Die oben beschriebenen drei Bewegungsrichtungen sind in Figur 1 veranschaulicht. Das Laparoskop ist hierbei mit dem Bezugszeichen 100, der Trokarpunkt mit dem Bezugszeichen 102 bezeichnet.

Um eine kontinuierliche Nachführung, also eine "glatte" Bewegung des Bildausschnittes zu erreichen, ist in der Regel eine Kombination aus einer PAN- und einer TILT-Bewegung erforderlich.

In den meisten Fällen erfolgt das Nachführen des Laparoskops manuell durch einen Assistenzarzt.

Alternativ zum manuellen Nachführen sind auch Haltevorrichtungen für Laparoskope bekannt, die in der Lage sind, die Nachführung des Laparoskops durchzuführen.

Die meisten solcher Haltesysteme sind sehr komplex aufgebaut, so dass selbst für das Durchführen einer einfachen TILT- oder ZOOM-mehrere Motoren für die Ansteuerung unterschiedlicher Gelenke gleichzeitig betätigt werden müssen. Insbesondere ist somit eine aufwendige Ansteuerung notwendig und es kann erforderlich sein, dass Interpolationen für die Ansteuerung verwendet werden.

Vorrichtung zum Halten und Bewegen eines Laparoskops sind beispielsweise aus den Dokumenten EP 0 761 177 B1, US 5 766 126 A, EP 2 169 346 B1, EP 2 520 244 A1, US 2010/168765 A1, EP 2 052 675 A1, US 2009/0112056 A1, US 6,932,089 B1, US 2006/0100501 A1, US 2011/0257475 A1, DE 103 05 693 B4, DE 10 2006 007 858 A1, DE 10 2007 019 363 B4, DE 10 2008 016 146 B4, EP 2 254 735 B1, US 2011/028992 A1, DE 10 2009 018 917 A1, EP 2 246 006 A2, EP 2 457 533 A2, US 5 976 156, US 6 024 695 A, DE 196 09 034 C2 und EP 2 008 605 A1 bekannt.

Das Dokument US 2007/0089557 A1 offenbart Antriebsstänge für Roboterarme mit einem mehrfach gefalteten Riemen.

Die US 2012/041263 A1 beschreibt eine zweiteilige endoskopische chirurgische Einrichtung.

Die US 2003/0221504 A1 betrifft ein entferntes Zentrum eines Bewegungsrobotersystems. Die US 2005/0234293 A1 offenbart eine Haltevorrichtung für eine endoskopische Einrichtung.

Es ist Aufgabe der Erfindung, eine Vorrichtung zum Halten und Bewegen eines Laparoskops während einer Operation anzugeben, die einfach und kompakt aufgebaut ist und leicht gesteuert werden kann.

Diese Aufgabe wird durch eine Vorrichtung mit den Merkmalen des Anspruchs 1 gelöst. Vorteilhafte Weiterbildungen der Erfindung sind in den abhängigen Ansprüchen angegeben.

Die Vorrichtung weist eine Befestigungseinheit zur Befestigung der Vorrichtung an einem Haltearm auf, der seinerseits wiederum insbesondere an einem Operationstisch befestigt ist. Ferner hat die Vorrichtung eine Aufnahmeeinheit zum Halten des Laparoskops, einen Grundkörper und einen Hebel, über den die Aufnahmeeinheit an dem Hauptkörper befestigt ist. Der Hauptkörper ist hierbei mit Hilfe einer ersten Rotationseinheit derart an der Befestigungseinheit gelagert, dass der Hauptkörper relativ zur Befestigungseinheit um eine erste Drehachse drehbar ist. Der Hebel seinerseits ist mit Hilfe einer zweiten Rotationseinheit derart an dem Hauptkörper befestigt, dass der Hebel um eine zur ersten Drehachse parallele zweite Drehachse relativ zum Hauptkörper drehbar ist. Ferner umfasst die Vorrichtung eine erste Antriebseinheit zum Antreiben der ersten und der zweiten Rotationseinheit, die bei Aktivierung die erste und zweite Rotationseinheit derart antreibt, dass der Hauptkörper um die erste Drehachse und der Hebel um die zweite Drehachse gedreht werden, wobei die Drehung des Hauptkörpers um die erste Drehachse und die Drehung des Hebels um die zweite Drehachse in entgegengesetzte Drehrichtung aber mit dem gleichen Drehwinkel erfolgen.

Hierdurch wird erreicht, dass der Aufhängungspunkt des Laparoskops auf einer Kreisbahn um den Trokarpunkt bewegt wird, so dass auf einfache Weise nur über die beiden Rotationseinheiten die Kippbewegung, also die TILT-Bewegung, des Laparoskops ausgeführt werden kann. Die Ausrichtung des Laparoskops wird hierbei durch den auf der Kreisbahn bewegten Lagerungspunkt und den Trokarpunkt selbst festgelegt. Die zuvor beschriebene Vorrichtung ermöglicht somit einen direkten Antrieb der TILT-Bewegung, d.h. dass diese TILT-Bewegung nicht aus mehreren Bewegungen zusammengesetzt werden muss, sondern ausschließlich durch die erste Antriebseinheit erfolgen kann.

Die Vorrichtung wird vor der Operation insbesondere derart ausgerichtet, dass der Drehpunkt der TILT-Bewegung dem Trokarpunkt entspricht.

Somit wird eine einfache elektronische Ansteuerung erreicht. Insbesondere ist keine Achseninterpolation notwendig.

Die erste Rotationseinheit umfasst insbesondere eine erste Welle, auf der die Befestigungseinheit drehfest gelagert ist. Entsprechend umfasst insbesondere auch die zweite Rotationseinheit eine zweite Welle, auf der der Hebel drehfest gelagert ist. Über die erste Antriebseinheit werden die erste und die zweite Welle immer um den gleichen Drehwinkel aber in entgegengesetzte Drehrichtung gedreht. Durch dieses synchrone entgegengesetzte Drehen um zwei parallele Achsen erfolgt auf einfache Weise die oben bereits beschriebene Führung des Befestigungspunktes des Laparoskops auf einer Kreisbahn um den Trokarpunkt, die die TILT-Bewegung ermöglicht.

Bei einer bevorzugten Ausführungsform der Erfindung umfasst die erste Antriebseinheit genau einen Motor, über den sowohl die erste als auch die zweite Rotationseinheit entsprechend der zuvor beschriebenen Art angetrieben sind. Hierzu ist insbesondere eine Königswelle vorgesehen, die von dem einen Motor angetrieben wird und die mit beiden Rotationseinheiten derart in Eingriff ist, dass diese in entgegengesetzte Richtung um den gleichen Drehwinkel gedreht werden. Die zuvor beschriebene Antriebseinheit hat den Vorteil, dass durch das Vorsehen nur eines Motors nur wenig Bauraum benötigt wird und somit der Hauptkörper sehr klein ausgebildet sein kann, was viel Platz für den Operateur lässt. Darüber hinaus bietet die rein mechanische Kopplung des einen Motors über die Königswelle mit beiden Rotationseinheiten die Sicherheit, dass immer eine synchrone Rotation stattfindet ohne dass hierfür aufwendige Sensoren und elektrische Ansteuerungen notwendig sind.

Bei der Königswelle handelt es sich vorzugsweise um eine teleskopierbare Königswelle, so dass, wie im Folgenden noch näher beschrieben wird, bei der Ausgestaltung einer Zoomfunktion über zwei linear zueinander fahrbare Gehäuseteile trotz der sich ändernden Distanz zwischen den beiden Rotationseinheiten über den einen Motor beide Rotationseinheiten sicher angetrieben werden können.

Es ist besonders vorteilhaft, wenn an den beiden Endbereichen der Königswelle jeweils eine Schneckenrad- oder Kegelradanordnung vorgesehen ist, mit der die Königswelle mit der ersten bzw. zweiten Rotationseinheit in Eingriff steht. Insbesondere ist an den Endbereichen der Königswelle jeweils eine Schnecke vorgesehen, die mit jeweils einem auf der ersten bzw. zweiten Welle der Rotationseinheiten angeordneten Schneckenrad in Eingriff steht. Auf diese Weise kann einfach und bei kompaktem Aufbau eine sichere Kraftübertragung gewährleistet werden. Ferner wird hierdurch auch eine Selbsthemmung erreicht.

Bei einer alternativen Ausführungsform der Erfindung können anstelle einer Königwelle auch ein Riementrieb oder ein Kettentrieb verwendet werden, wobei auch dieser insbesondere wieder Ausgleichselemente für die Ermöglichung der translatorischen ZOOM-Bewegung enthält. Insbesondere ist bei solchen Riemen- oder Kettentrieben ebenfalls eine Getriebestufe zwischengeschaltet, die die unterschiedliche Drehrichtung der beiden Rotationseinheiten umsetzt.

Ebenso ist alternativ eine hydraulische Kopplung des Motors mit den beiden Rotationseinheiten möglich.

Bei einer alternativen Ausführungsform der Erfindung kann die erste Antriebseinheit auch zwei Motoren, insbesondere zwei Schrittmotoren, umfassen, wobei einer dieser beiden Motoren zum Antreiben der ersten Rotationseinheit und der andere Motor zum Antreiben der zweiten Rotationseinheit dient. Ferner ist insbesondere eine Steuereinheit vorgesehen, welche die beiden Motoren derart ansteuert, dass diese die Rotationseinheiten synchron in entgegensetzte Richtungen drehen. Insbesondere sind Sensoren vorgesehen, die die Drehbewegung der beiden Rotationseinheiten erfassen und diese Signale bei der Ansteuerung der beiden Motoren entsprechend berücksichtigt werden. Somit können eventuell auftretende Schrittverluste bei Schrittmotoren kompensiert werden.

Die Aufnahmeeinheit ist insbesondere derart ausgebildet, dass das Laparoskop über sie frei drehbar an dem Hebel gelagert ist. Das Laparoskop kann sich insbesondere frei um eine Achse drehen, die parallel zu der ersten und der zweiten Drehachse verläuft. Somit wird eine Punktführung des Laparoskops erreicht. Bei einer solchen Punktführung wird lediglich der Aufhängepunkt des Laparoskops, wie zuvor beschrieben, über den entsprechenden TILT-Antrieb bewegt. Die Ausrichtung des Laparoskops während der Operation entsteht somit erst in dem Zusammenspiel mit dem Trokarpunkt, durch den das Laparoskop hindurchgeführt ist. Eine solche Punktführung hat den Vorteil, dass sie einfach mechanisch aufgebaut sein kann. Darüber hinaus hat sie den Vorteil, dass, wenn es zu Abweichungen zwischen dem virtuellen Drehpunkt, also dem Drehpunkt, um den das Laparoskop durch die Vorrichtung gedreht wird, und dem Trokarpunkt kommt, anders als bei einer Vektorführung nur geringe Kräfte durch die Bewegung des Laparoskops über das Laparoskop auf die Bauchdecke des Patienten ausgeübt werden, so dass Verletzungen des Patienten und/oder Beschädigungen des Laparoskops vermieden werden.

Bei einer alternativen Ausführungsform kann das Laparoskop auch über eine Vektorführung zwangsgeführt sein. In diesem Fall ist in dem Hebel insbesondere eine Parallelkurbel integriert, über die die Drehung des Laparoskops relativ zum Hebel gesteuert wird. Somit entsteht auf einfache Weise eine Vektorführung.

Bei einer besonders bevorzugten Ausführungsform der Erfindung weist der Hauptkörper der Vorrichtung ein Gehäuse auf, das ein erstes Gehäuseteil und ein von dem ersten Gehäuseteil getrenntes zweites Gehäuseteil umfasst. Die beiden Gehäuseteile sind insbesondere nicht direkt aneinander befestigt und aus einem harten, nur unwesentlich deformierbaren Material, beispielsweise Metall oder Kunststoff, gefertigt. Die erste Rotationseinheit ist insbesondere an dem ersten Gehäuseteil und die zweite Rotationseinheit an dem zweiten Gehäuseteil befestigt.

Ferner ist es vorteilhaft, wenn eine zweite Antriebseinheit vorgesehen ist, mit deren Hilfe das zweite Gehäuseteil relativ zum ersten Gehäuseteil in eine vorbestimmte Richtung entlang einer vorbestimmten Strecke linear bewegt werden kann. Über das Bewegen der beiden Gehäuseteile relativ zueinander wird der Abstand der beiden Rotationseinheiten und somit der Abstand zwischen der an ihnen gelagerten Befestigungseinheit und dem Hebel variiert. Hierüber wird somit das an dem Hebel befestigte Laparoskop entlang seiner Längsachse ohne Veränderung der Verkippung ebenfalls linear bewegt und somit in den Patienten tiefer hinein oder aus dem Patienten heraus bewegt. Somit kann auf einfache Weise eine ZOOM-Bewegung ausgeführt werden und der dargestellte Bildausschnitt vergrößert bzw. verkleinert werden.

Wie auch bei der TILT-Bewegung ist ein Direktantrieb gegeben, so dass auch die ZOOM-Bewegung nicht aus mehreren Einzelbewegungen zusammengesetzt werden muss, sondern ausschließlich durch ein Verstellen des Abstandes der beiden Gehäuseteile durch die zweite Antriebseinheit gewährleistet wird.

Die zweite Antriebseinheit umfasst insbesondere einen Motor, der eine Spindel antreibt, über die die beiden Gehäuseteile miteinander gekoppelt sind. Durch Drehen der Spindel wird je nach Drehrichtung das zweite Gehäuseteil auf das erste zubewegt oder von diesem entfernt.

Ferner ist es vorteilhaft, wenn das erste und das zweite Gehäuseteil über mindestens eine Linearführung, vorzugsweise über mehrere Linearführungen, miteinander verbunden sind. Über die Linearführungen wird erreicht, dass die beiden Gehäuseteile sicher aneinander befestigt sind und somit auch größere Kräfte übertragen werden können ohne dass es zu Verformungen, Verspannungen und/oder Beschädigungen kommt, so dass die gewünschte Ausrichtung des Laparoskops immer erhalten bleibt.

Die Linearführungen sind insbesondere in Form von zueinander verschiebbaren Schienen oder als in Kugelumlaufbüchsen gelagerte Rundführungen ausgebildet.

Ferner umfasst das Gehäuse vorzugsweise einen faltbaren Balg, der zwischen dem ersten und dem Gehäuseteil angeordnet ist. Somit wird erreicht, dass auch beim Verändern des Abstandes der beiden Gehäuseteile für die ZOOM-Bewegung ein geschlossenes Gehäuse jederzeit gegeben ist, so dass zum einen eine Beschädigung der innenliegenden Teile und zum anderen Verletzungen von Personen durch das Einklemmen zwischen den beiden Gehäuseteilen vermieden werden. Alternativ kann anstelle eines faltbaren Balges auch eine teleskopierbare Blechabdeckung verwendet werden.

Alternativ kann das erste Gehäuseteil auch zumindest teilweise auf das zweite Gehäuseteil oder das zweite Gehäuseteil zumindest teilweise auf das erste Gehäuseteil aufschiebbar ist. Hierdurch wird erreicht, dass das Gehäuse kein längenveränderbares Zwischenelement zwischen den beiden Gehäuseteilen benötigt, da trotz der für die ZOOM-Bewegungen notwendigen Längsverschiebungen nur durch die beiden Gehäuseteile immer ein geschlossenes Gehäuse gegeben ist, so dass zum einen eine Beschädigung der innenliegenden Teile und zum anderen Verletzungen von Personen durch das Einklemmen zwischen den beiden Gehäuseteilen vermieden werden. Ferner wird hierdurch eine Kraftübertragung direkt zwischen den beiden Gehäuseteilen möglich, so dass insbesondere auf die Linearführungen verzichtet werden kann.

Ferner ist es vorteilhaft, wenn die erste Antriebseinheit und die zweite Antriebseinheit, die erste Rotationseinheit, die zweite Rotationseinheit, die Linearführungen und/oder die Spindel innerhalb des Gehäuses des Hauptkörpers angeordnet sind. Somit wird zum einen ein sehr kompakter Aufbau erreicht und zum anderen ein Schutz dieser Bauteile gewährleistet. Insbesondere ragen ausschließlich die erste und die zweite Drehachse der Rotationseinheiten aus dem Gehäuse heraus, an denen dann die Befestigungseinheit bzw. der Hebel angeordnet sind.

Ferner ist es vorteilhaft, wenn die Befestigungseinheit einen ersten und einen zweiten Abschnitt umfasst, wobei der erste Abschnitt zwischen dem zweiten Abschnitt und dem Hauptkörper angeordnet ist und relativ zum zweiten Abschnitt um eine dritte Drehachse drehbar ist. Über das Drehen des ersten Abschnittes realtiv zum zweiten Abschnitt und somit das Drehen des Hauptkörpers relativ zum Haltearm erfolgt eine entsprechende Rotation des Laparoskops um die dritte Drehachse. Die Vorrichtung wird vor der Operation derart ausgerichtet, dass diese dritte Drehachse durch den Trokarpunkt hindurch verläuft und insbesondere orthogonal zur Ebene der Patientenlagerfläche angeordnet ist. Somit wird durch das Drehen um die dritte Drehachse die PAN-Bewegung ausgeführt.

Auch die PAN-Bewegung ist somit als Direktantrieb ausgebildet, d.h. dass auch hierfür nur die Ansteuerung einer Antriebseinheit zur Ausführung der Drehbewegung um die dritte Drehachse notwendig ist. Durch die Kombination von PAN- und TILT-Bewegung kann der Punkt, an dem das Laparoskop an dem Hebel drehbar gelagert ist, insgesamt auf einer kugelförmigen Fläche mit dem Trokarpunkt als Mittelpunkt der Kugel bewegt werden, so dass das Laparoskop durch die Führung im Trokarpunkt entsprechend verschwenkt und verkippt wird.

Es ist insbesondere eine dritte Antriebseinheit zum Drehen des ersten Abschnittes relativ zum zweiten Abschnitt vorgesehen, wobei auch die dritte Antriebseinheit insbesondere einen Motor umfasst. Dieser Motor ist insbesondere innerhalb des Gehäuses der Befestigungseinheit, vorzugsweise innerhalb des Gehäuses des ersten Abschnitts, angeordnet. Alternativ kann der Motor der dritten Antriebseinheit auch innerhalb des Gehäuses des Hauptkörpers angeordnet sein und über entsprechende Kopplungsanordnungen die Drehbewegung ausführen. Durch das Anordnen der dritten Antriebseinheit in der Befestigungseinheit und/oder dem Hauptkörper wird erreicht, dass auch die PAN-Bewegung ausschließlich von Teilen ausgeführt wird, die Teil der Vorrichtung sind. Somit kann der Haltearm, an dem die Vorrichtung befestigt wird, sehr einfach und rein passiv ausgebildet sein. Insbesondere muss dieser Haltearm keine elektrischen Bauteile umfassen, die die PAN-Bewegung ausführen.

Bei einer alternativen Ausführungsform kann die dritte Antriebseinheit in dem Haltearm angeordnet sein, was dann den Vorteil hat, dass die dritte Antriebseinheit nicht zusammen mit dem Hauptkörper gedreht werden muss. Ferner kann in diesem Fall die Verbindung zwischen dem Haltearm und dem Hauptkörper, als die Befestigungseinheit, besonders schlank, beispielsweise als schlanke Gabel oder einseitige Schwinge, ausgebildet sein.

Die dritte Drehachse, um die die PAN-Bewegung ausgeführt wird, ist insbesondere orthogonal zur ersten und zweiten Drehachse angeordnet.

Das Steuern des Bewegens des Laparoskops mit Hilfe der Vorrichtung erfolgt insbesondere mit Hilfe von Bildverarbeitungsprogrammen.

Zusätzlich kann an dem Hauptkörper insbesondere ein kleiner Joystick vorgesehen sein, über den, beispielsweise beim Ausfall des Bildsteuerungsverfahrens, ebenfalls eine Steuerung der Bewegung der Vorrichtung des Laparoskops erfolgen kann.

Weitere Merkmale und Vorteile der Offenbarung ergeben sich aus der folgenden Beschreibung, die die Offenbarung anhand von Ausführungsbeispielen im Zusammenhang mit den beigefügten Figuren näher erläutert.

Es zeigen:
- Figur 1: eine schematische Darstellung der drei Grundbewegungen eines Laparoskops während einer Operation;
- Figur 2: eine schematische Darstellung der allgemeinen Funktion einer erfindungsgemäßen Vorrichtung zum Halten und Bewegen eines Laparoskops;
- Figur 3: eine schematische perspektivische Darstellung einer Vorrichtung zum Halten und Bewegen eines Laparoskops gemäß einer ersten Ausführungsform;
- Figur 4: eine weitere schematische perspektivische Darstellung der Vorrichtung nach Figur 3 bei ausgeblendetem Balg;
- Figur 5: eine schematische perspektivische Darstellung der Vorrichtung nach Figuren 3 und 4 bei ausgeblendetem Gehäuse;
- Figur 6: eine schematische perspektivische Darstellung eines Ausschnitts der Vorrichtung nach den Figuren 3 bis 5;
- Figur 7: eine schematische perspektivische Darstellung einer Befestigungseinheit der Vorrichtung nach den Figuren 1 bis 6;
- Figur 8: eine schematische perspektivische Darstellung der Befestigungseinheit nach Figur 7 bei ausgeblendetem Gehäuse;
- Figur 9: eine schematische Darstellung eines Hauptkörpers einer Vorrichtung zum Halten und Bewegen eines Laparoskops gemäß einer zweiten Ausführungsform; und
- Figur 10: eine schematische perspektivische Darstellung einer Befestigungseinheit einer Vorrichtung zum Halten und Bewegen eines Laparoskops gemäß einer dritten Ausführungsform.

In Figur 1 ist eine schematische Darstellung eines auf einer Patientenlagerfläche 104 liegenden Patienten 106 während einer laparoskopischen Operation dargestellt. Das Laparoskop 100 ist durch einen Trokarpunkt 102 hindurch in die Bauchhöhle des Patienten 106 eingeführt. Hierbei werden mit Hilfe einer Kamera eines Laparoskops 100 Bilder des Operationsbereiches aufgenommen. Das Laparoskop 100 muss hierbei immer dem aktuellen Operationsbereich nachgeführt werden, insbesondere derart, dass der aktuell manipulierte Bereich im Bauchraum des Patienten 106 in der Mitte des auf dem nicht dargestellten Bildschirm gezeigten Bildes angeordnet ist.

Die Nachführbewegung lässt sich auf drei Grundbewegungen zurückführen. Zum einen kann das Laparoskop 100 um eine senkrecht zur Patientenlagerfläche 104, stehende z-Achse gedreht werden, was als PAN-Bewegung bezeichnet wird. Hierbei handelt es sich also um ein Schwenken des Laparoskops 100 in Richtung des Doppelpfeiles P1 um den Trokarpunkt 102.

Ferner kann das Laparoskop 100, wie durch den Pfeil P2 dargestellt, relativ zum Trokarpunkt 102 verkippt werden, was eine Rotation um eine in der xy-Ebene liegende Achse darstellt. Dieses Kippen des Laparoskops 100 wird als TILT-Bewegung bezeichnet.

Die dritte Grundbewegung ist die ZOOM-Bewegung, bei der das Laparoskop 100 entsprechend des Doppelpfeiles P3 in den Patienten 106 hinein oder hinaus bewegt wird, so dass ein Vergrößern bzw. Verkleinern des sichtbaren Bildausschnittes erfolgt. Somit erfolgt bei der ZOOM-Bewegung eine translatorische Bewegung des Laparoskops 100 entlang seiner eigenen Längsachse.

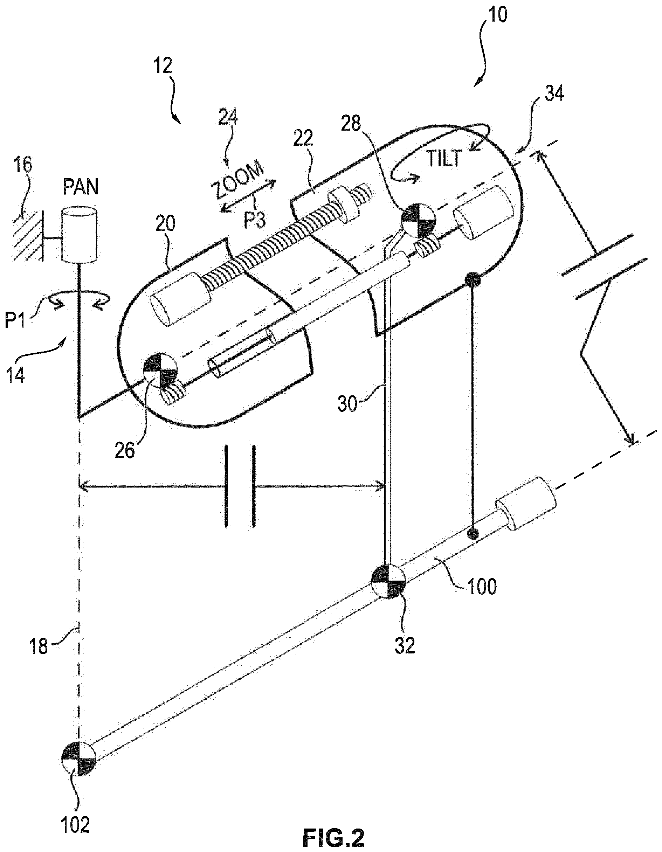

In Figur 2 ist eine rein schematische Darstellung der Funktionsweise einer erfindungsgemäßen Vorrichtung zum Halten und Bewegen eines Laparoskops 100 während einer Operation gezeigt.

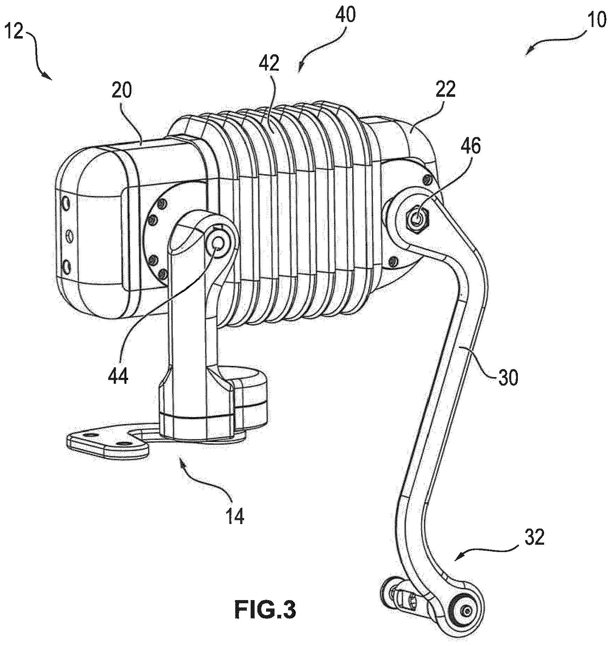

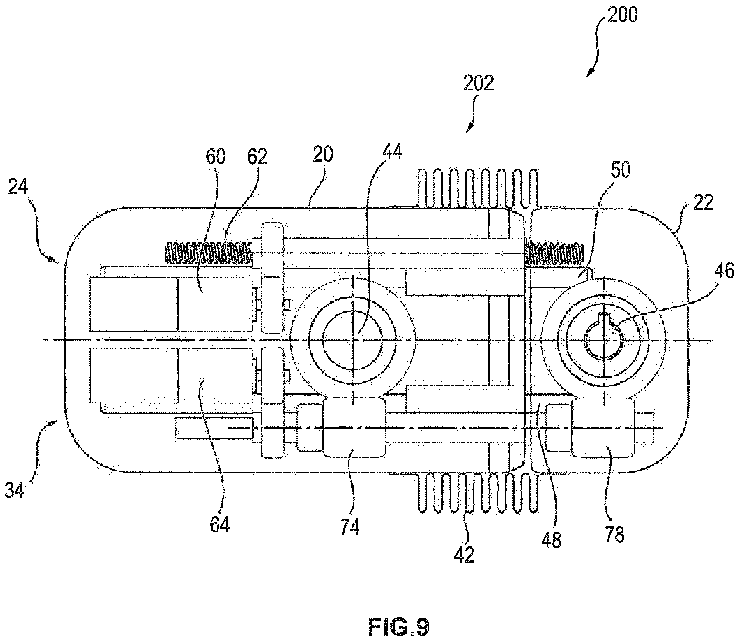

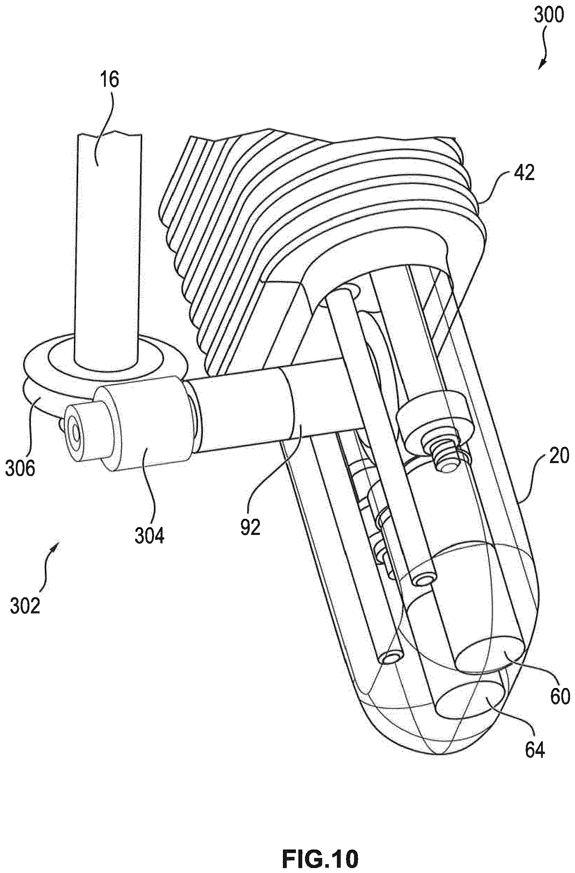

Die Figuren 3 bis 8 zeigen eine konkrete erste Ausführungsform, die Figur 9 eine zweite und die Figur 10 eine dritte Ausführungsform.

Die Vorrichtung 10 weist einen Hauptkörper 12 auf, der über eine Befestigungseinheit 14 an einem Haltearm 16 befestigt werden kann. Bei diesem Haltearm 16 handelt es sich insbesondere um eine Art Stativ, welches an seitliche Gleitschienen des Operationstisches befestigt werden kann.

Die Befestigungseinheit 14 ist hierbei derart ausgebildet, dass über sie die PAN-Bewegung realisiert wird, indem der Hauptkörper 12 über die Befestigungseinheit 14 relativ zum Haltearm 16 um eine dritte Drehachse 18 bewegt wird, welche durch den Trokarpunkt 102 verläuft und senkrecht zum Patienten 106 ausgerichtet ist.

Der Hauptkörper 12 weist zwei Gehäuseteile 20, 22 auf, die mit Hilfe einer zweiten Antriebseinheit 24 relativ zueinander linear in Richtung des Doppelpfeiles P3 verfahren werden können. Über die Einstellung des Abstandes zwischen den beiden Gehäuseteilen 20, 22 wird die ZOOM-Bewegung realisiert.

Der Hauptkörper 12 ist mit Hilfe einer ersten Rotationseinheit 28 relativ zur Befestigungseinheit 14 um eine erste Drehachse drehbar an der Befestigungseinheit 14 gelagert. Diese erste Rotationseinheit 26 ist hierbei an dem ersten Gehäuseteil 20 des Hauptkörpers 12 angeordnet.

An dem zweiten Gehäuseteil 22 ist eine zweite Rotationseinheit 26 vorgesehen, an der ein Hebel 30 befestigt ist. Die zweite Rotationseinheit 28 dient zum Drehen des Hebels 30 um eine zweite Drehachse, die parallel zur ersten Drehachse verläuft.

An dem der zweiten Rotationseinheit 28 entgegengesetzten Ende des Hebels 30 ist eine Aufnahmeeinheit 32 vorgesehen, an der das Laparoskop 100 befestigt ist. Diese Aufnahmeeinheit 32 ist derart ausgebildet, dass das Laparoskop 100 drehbar an dem Hebel 30 befestigt ist, so dass nur eine Punktführung erfolgt. Insbesondere ist durch die Aufnahmeeinheit 32 ein rotatorischer Freiheitsgrad freigegeben, der bei einer Zwangsführung nicht bestehen würde.

Ein weiterer Drehpunkt ist über den Trokarpunkt 102 gegeben, wobei sich die Ausrichtung des Laparoskops 100 somit immer über den Trokarpunkt 102 und die Position des Aufhängungspunktes der Aufnahmeeinheit 32 bestimmt. Somit wird eine reine Punktführung des Laparoskops 100 erreicht.

In dem Hauptkörper 12 ist eine erste Antriebseinheit 34 vorgesehen, welche die erste und die zweite Rotationseinheit 26, 28 antreibt. Hierzu ist die erste Antriebseinheit 34 derart ausgebildet, dass sie die erste und die zweite Rotationseinheit 28 jeweils um den gleichen Drehwinkel, aber in entgegengesetzte Richtungen dreht. Somit wird der Hauptkörper 12 relativ zur Befestigungseinheit 14 und der Hebel 30 relativ zum Hauptkörper 12 immer um den gleichen Winkel aber in entgegengesetzte Richtungen gedreht. Dies bewirkt, dass, sofern keine PAN-Bewegung ausgeführt wird, der Aufhängungspunkt des Laparoskops 100 an dem Hebel 30 auf einer kreisförmigen Bahn bewegt wird, durch die die TILT-Bewegung ausgeführt wird. Durch Betätigen der PAN-Bewegung kann somit insgesamt der Aufhängungspunkt nicht nur auf einer Kreisbahn, sondern auf einer kugelförmigen Fläche um den Trokarpunkt 102 bewegt werden.

Somit erfolgt die TILT-Bewegung ausschließlich durch die erste Antriebseinheit 34 und das entsprechende synchron entgegengesetzte Antreiben der Rotationseinheit 26, 28. Die ZOOM-Bewegung wird ausschließlich durch den sich verändernden Abstand der beiden Gehäuseteile 20, 22 durch die zweite Antriebseinheit 24 erreicht. Die PAN-Bewegung dagegen wird ausschließlich durch das Drehen des Hauptkörpers 12 um die dritte Drehachse 18 mit Hilfe der Befestigungseinheit 14 erreicht.

Somit wird insgesamt ein Direktantrieb erreicht, bei dem jede der drei Grundbewegungen über eine eigene Antriebseinheit 24, 34, 90 sehr einfach ausgeführt werden kann. Insbesondere sind keine aufwendigen Ansteuerungen notwendig und es müssen keine Kurveninterpolationen durchgeführt werden. Somit kann die Steuerung über ein Bildverarbeitungsprogramm erfolgen, ohne dass Sensor zur Erfassung der aktuellen Position und Ausrichtung notwendig sind.

In Figur 3 ist eine schematische perspektivische Darstellung einer Vorrichtung 10 zum Halten und Bewegen eines Laparoskops 100 während einer Operation gemäß einer ersten Ausführungsform dargestellt. Der Hauptkörper 12 weist ein Gehäuse 40 auf, das ein erstes Gehäuseteil 20, ein zweites Gehäuseteil 22 sowie einen zwischen dem ersten und dem zweiten Gehäuseteil angeordneten faltbaren Balg 42 umfasst.

Aus dem ersten Gehäuseteil 20 ragt eine erste Welle 44 der ersten Rotationseinheit 26 heraus, auf der die Befestigungseinheit 14 drehfest gelagert ist.

Ebenso ragt aus dem zweiten Gehäuseteil 22 eine zweite Welle 46 der zweiten Rotationseinheit 28 heraus, auf der wiederum der Hebel 30 drehfest gelagert ist. Die Aufnahmeeinheit 32 zum Befestigen des Laparoskops 100 ist hierbei nur rein schematisch dargestellt. Das Laparoskop 100 selbst ist nicht gezeigt.

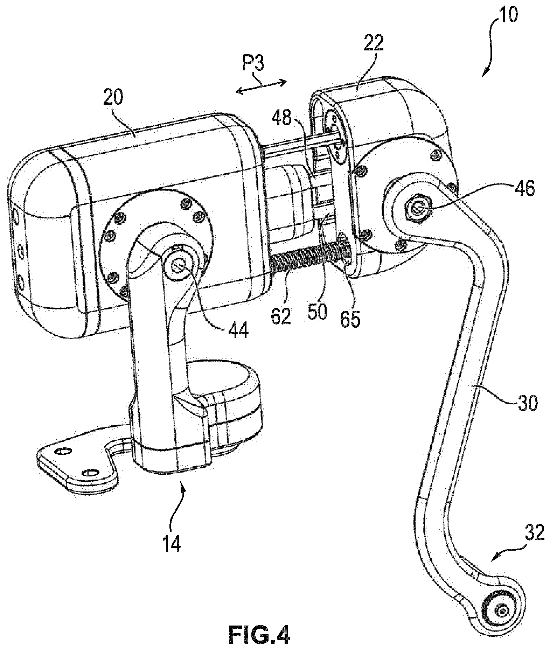

In Figur 4 ist eine weitere, schematische, perspektivische Darstellung der Vorrichtung 10 nach Figur 3 gezeigt, wobei der Balg 42 ausgeblendet ist. Wie nun zu erkennen ist, sind die beiden Gehäuseteile 20, 22 vollständig voneinander getrennt und können entsprechend des Doppelpfeiles P3 aufeinander zu und voneinander weg bewegt werden. Je nach Abstand zwischen den beiden Gehäuseteilen 20, 22 ändert sich auch der Abstand der beiden Wellen 44, 46 und somit der Abstand zwischen dem Hebel 30 und der Befestigungseinheit 14. Dies wiederum hat zur Folge, dass durch Ändern des Abstandes der beiden Gehäuseteile 20, 22 zueinander das Laparoskop 100 translatorisch in Richtung seiner Längsachse bewegt wird, so dass eine ZOOM-Bewegung ausgeführt wird.

Die beiden Gehäuseteile 20, 22 sind über zwei Linearführungen 48, 50 miteinander verbunden, die das lineare Verschieben der beiden Gehäuseteile 20, 22 zueinander ermöglichen und dennoch eine Kraftübertragung gewährleisten, so dass die auf das Laparoskop 100 wirkenden Kräfte auf den Haltearm übertragen werden können und ein Verkanten verhindert wird.

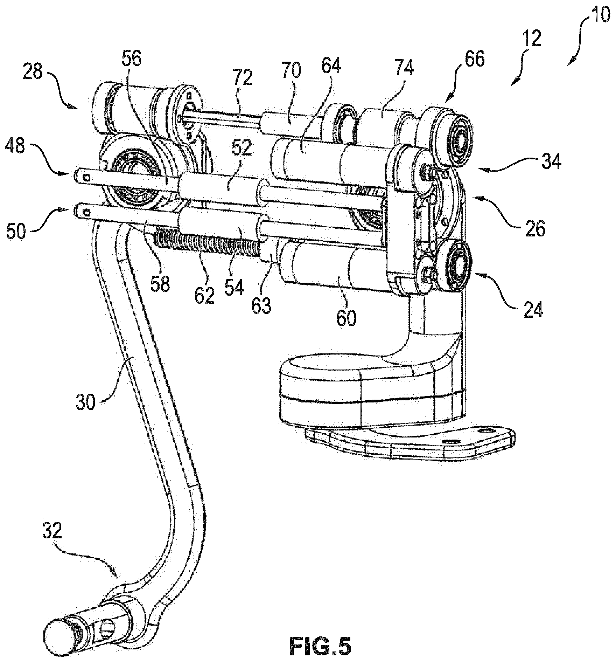

In Figur 5 ist eine weitere, schematische, perspektivische Darstellung der Vorrichtung nach den Figuren 3 und 4 gezeigt, wobei hierbei das Gehäuse 40 vollständig ausgeblendet ist, um die innerhalb des Gehäuses 40 liegenden Bauteile, insbesondere die erste und die zweite Antriebseinheit 34, 24 sichtbar zu machen. Hierbei ist bei Figur 5 der Blick, verglichen zu den Figuren 3 und 4 auf die entgegengesetzte Seite gerichtet.

Die Linearführungen 48, 50 bestehen jeweils aus einer Kugelbüchse 52, 54 in der Rundführungen 56, 58 geführt sind.

Die zweite Antriebseinheit 24 weist einen Motor 60 auf, über den eine Spindelmutter 63 angetrieben werden kann. Eine Spindel 62 ist auf der Seite des ersten Gehäuseteiles 20 in der Spindelmutter 63 gelagert. Das entgegengesetzte Ende der Spindel 62 ist in einer Aussparung 65 des zweiten Gehäuseteiles 22 gelagert. Durch Drehen der Spindelmutter 63 wird, in Abhängigkeit der Drehrichtung der Spindel 62, das zweite Gehäuseteil 22 auf das erste Gehäuseteil 20 zu bewegt oder von dem ersten Gehäuseteil 20 weg bewegt.

Die erste Antriebseinheit 34 zum Antreiben der Rotationseinheiten 26, 28 umfasst einen Motor 64, der über eine Zahnradanordnung 66 eine Königswelle 68 antreibt. Die Königswelle 68 ist hierbei teleskopierbar ausgebildet, d.h. sie umfasst zwei Wellen 70, 72, die relativ zueinander linear verfahrbar und drehfest sind. Somit kann über einen Motor 64 ein Antreiben der in den verschiedenen Gehäuseteilen 20, 22 angeordneten Rotationseinheiten 26, 28 erfolgen und dennoch eine Verstellung des Abstandes der Gehäuseteile 20, 22 gewährleistet werden.

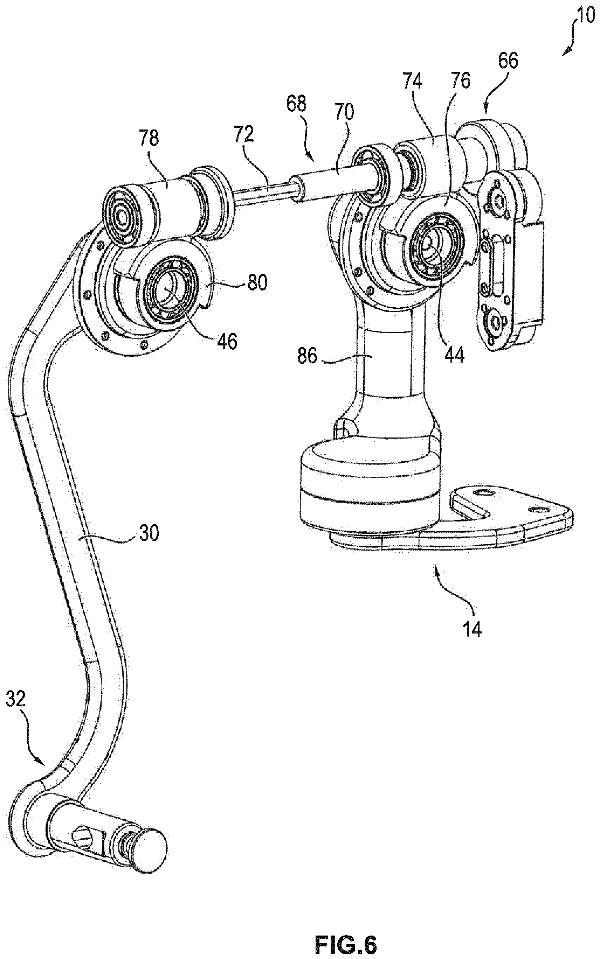

Auf der Königswelle 68 ist eine erste Schnecke 74 angeordnet, die wie in Figur 6 gezeigt ist, mit einem ersten Schneckenrad 76 in Eingriff ist. Das Schneckenrad 76 ist auf der ersten Welle 44 drehfest gelagert.

Ferner ist auf der Königswelle 68 eine zweite Schnecke 78 angeordnet, die mit einem zweiten Schneckenrad 80 in Eingriff ist, welches wiederum drehfest auf der zweiten Welle 46 der zweiten Rotationseinheit 28 gelagert ist.

Die Schnecken 74, 78 und die Schneckenräder 76, 80 sind hierbei derart ausgebildet, dass das Drehen der Wellen 44, 46 beim Drehen der Königswelle 68 über den Motor 64 um den gleichen Drehwinkel in entgegengesetzter Drehrichtung erfolgt. Hierdurch wird auf einfache Weise die im Zusammenhang mit Figur 2 beschriebene für die TILT-Bewegung erforderliche Führung des Aufhängungspunktes des Laparoskops 100 auf einer Kreisbahn erreicht.

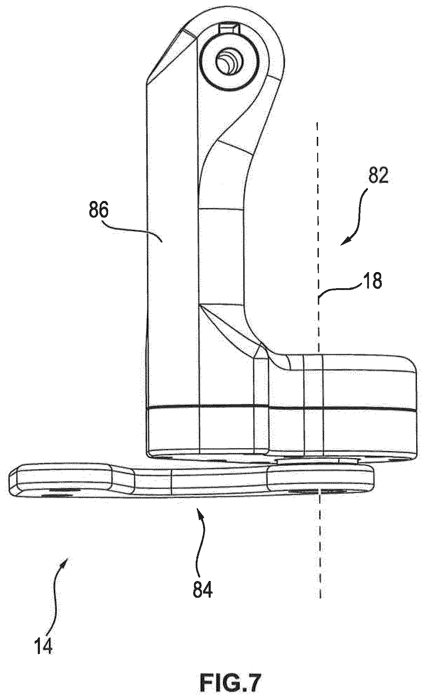

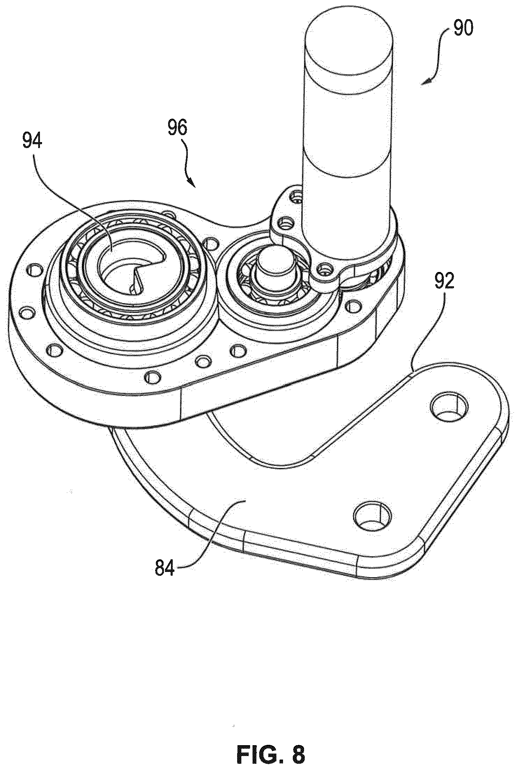

In Figur 7 ist eine schematische perspektivische Darstellung der Befestigungseinheit 14 gezeigt. Die Befestigungseinheit 14 weist einen ersten Abschnitt 82 sowie einen zweiten Abschnitt 84 auf, wobei der zweite Abschnitt 84 derart ausgebildet ist, dass er über einen nicht gezeigten Schnellverschluss an einem Haltearm 16 befestigt werden kann. Die beiden Abschnitte 82, 84 sind derart miteinander verbunden, dass sie relativ zueinander um die dritte Drehachse 18 gedreht werden können. Hierzu ist innerhalb des Gehäuses 86 des ersten Abschnittes 82 eine dritte Antriebseinheit 90 vorgesehen, die in Figur 8 gezeigt ist. Die dritte Antriebseinheit 90 weist einen Motor 92 auf, mit dessen Hilfe eine Welle 94 gedreht werden kann, die drehfest mit dem zweiten Abschnitt 84 verbunden ist. Hierzu ist der Motor 90 über eine Zahnradanordnung 96 mit der Welle 94 verbunden. Die dritte Antriebseinheit 90 ist hierbei insbesondere vollständig wälzgelagert. Die Verbindung mit der Abtriebswelle des Motors 92 wird insbesondere über eine Stirnradkaskade hergestellt. In Figur 9 ist eine schematische Darstellung eines Hauptkörpers 202 einer Vorrichtung 200 gemäß einer zweiten Ausführungsform dargestellt. Elemente mit gleichem Aufbau oder gleicher Funktion haben dieselben Bezugszeichen wie bei der ersten Ausführungsform. Im Unterschied zu der Ausführung nach den Figuren 3 bis 8 sind hierbei die Motoren 60, 64 der ersten und der zweiten Antriebseinheit 34, 24 um 180° gedreht angeordnet.

Die Ausführungsform nach den Figuren 3 bis 8 hat jedoch den Vorteil, dass diese kompakter ausgebildet ist.

In Figur 10 ist eine schematische Darstellung eines Ausschnitts einer Vorrichtung 300 gemäß einer dritten Ausführungsform gezeigt, wobei hierbei insbesondere die Befestigungseinheit 302 anders ausgebildet ist. Die Befestigungseinheit umfasst eine über den Motor 92 angetriebene Schnecke 304, die mit einem Schneckenrad 306 in Eingriff ist.

Darüber hinaus ist hierbei der Motor 92 im Unterschied zur ersten Ausführungsform zumindest teilweise auch innerhalb des Gehäuses 40 des Hauptkörpers 12 angeordnet.

An der Befestigungseinheit 14, 302 kann insbesondere jeweils ein Laser vorgesehen sein, mit Hilfe dessen die Ausrichtung auf den Trokarpunkt 102 erfolgen kann. Alternativ kann die Ausrichtung beispielsweise auch über eine separate Einstelllehre erfolgen.

Der Hebel 30 ist insbesondere derart ausgebildet, dass er keine inneren Bauteile enthält und somit sehr einfach ausgebildet ist. Somit kann der Hebel als Einwegartikel oder aus einem sterilisierbaren Material ausgeführt sein.

Der Hebel 30 ist insbesondere S-förmig ausgebildet, wodurch dem Operateur möglichst viel Platz geschaffen wird, um das Laparoskop 100 bei Bedarf möglichst gut um seine Längsachse drehen zu können.

Alle zuvor beschriebenen Vorrichtungen 10, 200, 300 haben den Vorteil, dass jede der Bewegungen, also die PAN-Bewegung, die TILT-Bewegung und die ZOOM-Bewegung, durch eine eigene Antriebseinheit 24, 34, 90 angetrieben wird. Somit kommt die Vorrichtung 10, 200, 300 ohne Sensorik aus, was zu einer Erhöhung der Ausfallsicherheit führt. Darüber hinaus ist die Ansteuerung besonders einfach, da jeweils nur eine Antriebseinheit 24, 34, 90 für die Grundbewegungen angesteuert werden muss. Darüber hinaus fallen geringe Herstellungskosten an.

Ferner haben die Vorrichtungen 10, 200, 300 den Vorteil, dass sie kompakt und vergleichsweise leicht aufgebaut sind. Sie können somit problemlos an die Gleitschienen des Operationstisches angebracht werden, so dass die Verstellung des Operationstisches möglich ist, ohne dass ein Nachjustieren der Vorrichtungen 10, 200, 300 erforderlich wäre.

Darüber hinaus sind die Vorrichtungen 10, 200, 300 unabhängig von dem Haltearm, d.h., dass alle für die Bewegung des Laparoskops 100 notwendigen Komponenten in der Vorrichtung 10 selbst angeordnet sind. Somit können die einzelnen Komponenten leicht gehandhabt werden, da sie nacheinander an den Operationstisch montiert werden können. Somit kann das System von nur einer Person angebracht werden und hat ein geringes Packmaß.

Darüber hinaus ist der Aufwand für die anfängliche Ausrichtung auf den Trokarpunkt 102 sehr gering. Es ist, anders als bei vielen bekannten Systemen, keine aufwendige Sensorik notwendig, die durch Referenzfahrten initialisiert werden muss.

Durch die Punktführung und die gelenkige Aufhängung des Laparoskops 100 an dem Hebel 30 können Fehler bis hin zu einigen Zentimetern bei der Ausrichtung auf den Trokarpunkt 102 toleriert werden, ohne dass es zu Verletzungen des Patienten und/oder Beschädigungen der Vorrichtungen 10, 200, 300 und/oder des Laparoskops 100 kommt.

Darüber hinaus weisen die Vorrichtungen 10, 200, 300 nur eine sehr geringe Störkontur auf. Um den Trokarpunkt 102 herum besteht viel Bewegungsfreiheit für das Einbringen der Arbeitstokare und die Bewegung der laparoskopischen Instrumente. Dies wird insbesondere durch den Einsatz eines sehr schlanken Aufhängearms zur Fixierung der Optik erreicht. Des Weiteren kann dieser Arm als sterile Einwegkomponente oder als steriles Teil ausgeführt werden, so dass kein störender Sterilüberzug im Arbeitsbereich direkt über den Operationsbereich erforderlich ist.

Darüber hinaus erfordern die Vorrichtungen 10, 200, 300 keine außenliegenden Kabel im sterilen Bereich.

Die mittige Ausrichtung der Vorrichtungen 10, 200, 300 über den Patienten 106 führt dazu, dass der Operateur in seiner Bewegungsfreiheit nur minimal eingeschränkt wird. Da der Bereich direkt über der Bauchdecke des Patienten 106 frei von mechanischen Elementen ist, ist ein Zurückziehen der Trokarhülse ohne Einschränkung oder Kollisionen möglich.

Die gelenkige Aufhängung des Laparoskops 100 an dem Hebel 30 führt zu einer Punktführung, die wiederum das Risiko für mechanische Verspannungen und somit das Auftreten von Zwangskräften, die auf die Bauchdecke des Patienten 106 oder das eingespannte Laparoskop 100 wirken können, minimiert. Hierdurch wird die Patientensicherheit erhöht und das Risiko von Beschädigungen an dem Laparoskop 100 reduziert.

Darüber hinaus sind die Vorrichtungen 10, 200, 300 kompakt aufgebaut und es werden durch das allseitig geschlossene Gehäuse 40 Quetsch- und Scherstellen minimiert und eine gute Reinigbarkeit ermöglicht.

Das gekröpfte Design des Hebels 30, über den das Laparoskop 100 befestigt ist, erlaubt einen sehr großen Winkelbereich für die Rotation des Laparoskops 100.

Darüber hinaus sind die Vorrichtungen 10, 200, 300 durch die Verwendung von Schneckengetrieben 74, 76, 78, 80 selbsthemmend, so dass auf Bremsen verzichtet werden kann. Ferner wird die Sicherheit bei einem Unterbrechen der Energieversorgung erhöht, da die Schneckengetriebe im stromlosen Zustand automatisch ein Bewegen verhindern.

### Bezugszeichenliste

- 10, 200, 300: Vorrichtung
- 12: Hauptkörper
- 14: Befestigungseinheit
- 16: Haltearm
- 18: dritte Drehachse
- 20, 22: Gehäuseteil
- 24, 34, 90: Antriebseinheit
- 26, 28: Rotationseinheit
- 30: Hebel
- 32: Aufnahmeeinheit
- 40: Gehäuse
- 42: Balg
- 44, 46: Welle
- 48, 50: Linearführung
- 52, 54: Kugelumlaufbüchse
- 56, 58: Rundführung
- 60, 64, 92: Motor
- 62: Spindel
- 63: Spindelmutter
- 65: Aussparung
- 66: Zahnradanordnung
- 68: Königswelle
- 70, 72: Welle
- 74, 78: Schnecke
- 76, 80: Schneckenrad
- 82, 84: Abschnitt
- 86: Gehäuse
- 94: Welle
- 96: Zahnradanordnung
- 100: Laparoskop
- 102: Trokarpunkt
- 104: Patientenlagerfläche
- 106: Patient
- 202: Hauptkörper
- 302: Befestigungseinheit
- 304: Schnecke
- 306: Schneckenrad
- P1, P2, P3: Richtung

## Patentansprüche

1. Vorrichtung zum Halten und Bewegen eines Laparoskops während einer Operation
mit einer Befestigungseinheit (14, 302) zur Befestigung der Vorrichtung (10, 200, 300) an einem Haltearm (16),
einer Aufnahmeeinheit (32) zum Halten des Laparoskops (100),
einem Hauptkörper (12, 202), und
mit einem Hebel (30), über den die Aufnahmeeinheit (32) an dem Hauptkörper (12, 202) befestigt ist,
wobei der Hauptkörper (12, 202) über eine erste Rotationseinheit (26) um eine erste Drehachse drehbar an der Befestigungseinheit (14, 302) gelagert ist,
wobei der Hebel (30) über eine zweite Rotationseinheit (28) um eine zur ersten Drehachse parallele, zweite Drehachse drehbar an dem Hauptkörper (12, 202) befestigt ist, und
wobei eine erste Antriebseinheit (34) vorgesehen ist, die bei Aktivierung die erste und die zweite Rotationseinheit (26, 28) derart antreibt, dass der Hauptkörper (12, 202) um die erste Drehachse und der Hebel (30) um die zweite Drehachse um den gleichen Drehwinkel in entgegengesetzte Drehrichtungen gedreht werden,
**dadurch gekennzeichnet, dass** der Hauptkörper (12, 202) ein Gehäuse (40) aufweist, das ein erstes Gehäuseteil (20) und ein von diesem ersten Gehäuseteil (20) getrenntes zweites Gehäuseteil (22) umfasst, und dass die erste Rotationseinheit (26) an dem ersten Gehäuseteil (20) und die zweite Rotationseinheit (28) an dem zweiten Gehäuseteil (22) gelagert ist, und
dass eine zweite Antriebseinheit (24) vorgesehen ist, mit Hilfe derer das zweite Gehäuseteil (22) relativ zum ersten Gehäuseteil (20) entlang einer vorbestimmten Strecke linear bewegbar ist.

2. Vorrichtung (10, 200, 300) nach Anspruch 1, **dadurch gekennzeichnet, dass** die erste Rotationseinheit (26) eine erste Welle (44) umfasst, auf der die Befestigungseinheit (14, 302) drehfest gelagert ist, dass die zweite Rotationseinheit (28) eine zweite Welle (46) umfasst, auf der der Hebel (30) drehfest gelagert ist, und dass die erste und die zweite Welle (44, 46) über die erste Antriebseinheit (34) in entgegengesetzte Drehrichtungen um den gleichen Drehwinkel gedreht werden.

3. Vorrichtung (10, 200, 300) nach Anspruch 1 oder 2, **dadurch gekennzeichnet, dass** die erste Antriebseinheit (34) genau einen Motor (64) umfasst, über den die erste und die zweite Rotationseinheit (26, 28) angetrieben sind, wobei vorzugsweise die erste Antriebseinheit (34) eine Königswelle (68), insbesondere eine teleskopierbare Königswelle (68), umfasst, die über den Motor (64) angetrieben ist, und vorzugsweise die Königswelle (68) die beiden Rotationseinheiten (26, 28) antreibt.

4. Vorrichtung (10, 200, 300) nach Anspruch 3, **dadurch gekennzeichnet, dass** die Königswelle (68) über eine erste Schneckenradanordnung (74, 76) oder Kegelradanordnung mit der ersten Welle (44) der ersten Rotationseinheit (26) in Eingriff steht, und dass die Königswelle (68) über eine zweite Schneckenradanordnung (78, 80) oder Kegelradanordnung mit der zweiten Welle (46) der zweiten Rotationseinheit (28) in Eingriff steht.

5. Vorrichtung (10, 200, 300) nach Anspruch 3, **dadurch gekennzeichnet, dass** der Motor (64) über Ketten- und/oder Riementriebe und/oder hydraulische Kopplungen die Rotationseinheiten (26, 28) antreibt.

6. Vorrichtung (10, 200, 300) nach Anspruch 1 oder 2, **dadurch gekennzeichnet, dass** die erste Antriebseinheit (34) zwei Motoren umfasst, wobei jeweils ein Motor zum Antreiben einer Rotationseinheit (26, 28) dient, und dass eine Steuereinheit vorgesehen ist, die die beiden Motoren synchron ansteuert.

7. Vorrichtung (10, 200, 300) nach einem der vorhergehenden Ansprüche, **dadurch gekennzeichnet, dass** die Aufnahmeeinheit (32) derart ausgebildet ist, dass das Laparoskop (100) über sie frei drehbar an dem Hebel (30) gelagert ist.

8. Vorrichtung (10, 200, 300) nach Anspruch 1, **dadurch gekennzeichnet, dass** die zweite Antriebseinheit (24) einen Motor (60) und eine über diesen angetriebene Spindelmutter (63) umfasst, und dass das erste und das zweite Gehäuseteil (20, 22) über eine in der Spindelmutter (63) geführte Spindel (62) miteinander gekoppelt sind.

9. Vorrichtung (10, 200, 300) nach Anspruch 1 oder 8, **dadurch gekennzeichnet, dass** das erste und das zweite Gehäuseteil (20, 22) über mindestens eine Linearführung (48, 50) miteinander verbunden sind.

10. Vorrichtung (10, 200, 300) nach Anspruch 1, **dadurch gekennzeichnet, dass** das Gehäuse (40) einen faltbaren Balg (42) und/oder eine teleskopierbare Blechabdeckung umfasst, der bzw. die zwischen dem ersten und dem zweiten Gehäuseteil (20, 22) angeordnet ist.

11. Vorrichtung (10, 200, 300) nach Anspruch 1, **dadurch gekennzeichnet, dass** das erste Gehäuseteil (20) zumindest teilweise auf das zweite Gehäuseteil (22) oder das zweite Gehäuseteil (22) zumindest teilweise auf das erste Gehäuseteil (20) schiebbar ist.

12. Vorrichtung (10, 200, 300) nach Anspruch 1, **dadurch gekennzeichnet, dass** die erste Antriebseinheit (34), die zweite Antriebseinheit (24), die erste Rotationseinheit (26) und/oder die zweite Rotationseinheit (28) jeweils zumindest teilweise, vorzugsweise vollständig, innerhalb des Gehäuses (40) angeordnet sind.

13. Vorrichtung (10, 200, 300) nach einem der vorhergehenden Ansprüche, **dadurch gekennzeichnet, dass** die Befestigungseinheit (14, 302) einen ersten Abschnitt (82) und einen zweiten Abschnitt (84) umfasst, und dass der erste Abschnitt (82) relativ zum zweiten Abschnitt (84) um eine dritte Drehachse (18) drehbar ist, wobei vorzugsweise eine dritte Antriebseinheit (90) zum Drehen des ersten Abschnitts (82) relativ zum zweiten Abschnitt (84) vorgesehen ist, wobei bevorzugt die dritte Antriebseinheit (90) zumindest teilweise, vorzugsweise vollständig, in dem Gehäuse (40) des Hauptkörpers (12, 202) angeordnet ist oder alternativ die dritte Antriebseinheit (90) zumindest teilweise, vorzugsweise vollständig, in einem Gehäuse (86) der Befestigungseinheit (14, 302) angeordnet ist.

14. Vorrichtung (10, 200, 300) nach Anspruch 13, **dadurch gekennzeichnet, dass** die dritte Drehachse (18) orthogonal zur ersten und zweiten Drehachse angeordnet ist.

15. Vorrichtung (10, 200, 300) nach einem der Ansprüche 13 bis 14, **dadurch gekennzeichnet, dass** die Vorrichtung (10, 200, 300) einen Haltearm (16) zur Befestigung der Vorrichtung (10, 200, 300) an einem Operationstisch umfasst, und dass die dritte Antriebseinheit (90) innerhalb dieses Haltearms (16) angeordnet ist.

## Claims

1. A device for holding and moving a laparoscope during an operation
comprising a fastening unit (14, 302) for fastening the device (10, 200, 300) on a holding arm (16),
a receiving unit (32) for holding the laparoscope (100),
a main body (12, 202), and
comprising a lever (30), by means of which the receiving unit (32) is attached to the main body (12, 202),
wherein the main body (12, 202) is mounted on the fastening unit (14, 302) by means of a first rotational unit (26) to be rotatable about a first axis of rotation,
wherein the lever (30) is attached to the main body (12, 202) by means of a second rotational unit (28) to be rotatable about a second axis of rotation parallel to the first axis of rotation, and
wherein a first drive unit (34) is provided, which, when activated, drives the first and second rotational units (26, 28), so that the main body (12, 202) is rotated about the first axis of rotation, and the lever (30) is rotated about the second axis of rotation, in opposite directions of rotation by the same angle of rotation,
**characterized in that** the main body (12, 202) has a housing (40), comprising a first housing part (20) and a second housing part (22), which is separate from said first housing part (20), and the first rotational unit (26) is mounted on the first housing part (20), and the second rotational unit (28) is mounted on the second housing part (22), and
a second drive unit (24), with the help of which the second housing part (22) can be moved linearly along a predetermined segment in relation to the first housing part (20), is provided.

2. The device (10, 200, 300) according to claim 1, **characterized in that** the first rotational unit (26) comprises a first shaft (44) on which the fastening unit (14, 302) is in a rotationally fixed mount; the second rotational unit (28) comprises a second shaft (46), on which the lever (30) is in a fixed rotational mount; and the first and second shafts (44, 46) are rotated in opposite directions of rotation by the same angle of rotation by means of the first drive unit (34).

3. The device (10, 200, 300) according to claim 1 or 2, **characterized in that** the first drive unit (34) comprises exactly one motor (64), by means of which the first and second rotational units (26, 28) are driven, wherein the first drive unit (34) preferably comprises a master shaft (68), in particular a telescoping master shaft (68) driven by the motor (64), and said master shaft (68) preferably drives the two rotational units (26, 28).

4. The device (10, 200, 300) according to claim 3, **characterized in that** the master shaft (68) engages with the first shaft (64) of the first rotational unit (26) by means of a first worm gear configuration (74, 76) or bevel gear configuration, and the master shaft (68) engages with the second shaft (46) of the second rotational unit (28) by means of a second worm gear configuration (78, 80) or bevel gear configuration.

5. The device (10, 200, 300) according to claim 3, **characterized in that** the motor (64) drives the rotational units (26, 28) by means of chain drives and/or belt drives and/or hydraulic couplings.

6. The device (10, 200, 300) according to claim 1 or 2, **characterized in that** the first drive unit (34) comprises two motors, wherein one motor each is used to drive a rotational unit (26, 28), and one control unit is provided to control the two motors in synchronization.

7. The device (10, 200, 300) according to any one of the preceding claims, **characterized in that** the receiving unit (32) is designed so that the laparoscope (100) is mounted to be freely rotatable on the lever (30) by means of the receiving unit.

8. The device (10, 200, 300) according to claim 1, **characterized in that** the second drive unit (24) comprises a motor (60) and a spindle nut (63) driven by said motor, and the first and second housing parts (20, 22) are coupled to one another by means of a spindle (62) that is guided in the spindle nut (63).

9. The device (10, 200, 300) according to claim 1 or 8, **characterized in that** the first and second housing parts (20, 22) are connected to one another by means of at least one linear guide (48, 50).

10. The device (10, 200, 300) according to claim 1, **characterized in that** the housing (40) comprises folding bellows (42) and/or a telescoping sheet metal cover, which is situated between the first and second housing parts (20, 22).

11. The device (10, 200, 300) according to claim 1, **characterized in that** the first housing part (20) is displaceable at least partially onto the second housing part (22), or the second housing part (22) is displaceable at least partially onto the first housing part (20).

12. The device (10, 200, 300) according to claim 1, **characterized in that** the first drive unit (34), the second drive unit (24), the first rotational unit (26) and/or the second rotational unit (28) are each arranged at least partially, preferably completely, inside the housing (40).

13. The device (10, 200, 300) according to any one of the preceding claims, **characterized in that** the fastening unit (14, 302) comprises a first section (82) and a second section (84), and the first section (82) can be rotated about the third axis of rotation (18) relative to the second section (84), wherein the third drive unit (90) is preferably provided for rotation of the first section (82) relative to the second section (84), wherein the third drive unit (90) is situated at least partially, preferably completely, in the housing (40) of the main body (12, 202), or alternatively, the third drive unit (90) is situated at least partially, preferably completely, in a housing (86) of the fastening unit (14, 302).

14. The device (10, 200, 300) according to claim 13, **characterized in that** the third axis of rotation (18) is situated orthogonally to the first and second axes of rotation.

15. The device (10, 200, 300) according to any one of claims 13 to 14, **characterized in that** the device (10, 200, 300) comprises a holding arm (16) for fastening the device (10, 200, 300) on an operating table, and the third drive unit (90) is situated inside this holding arm (16).

## Revendications

1. Dispositif destiné à tenir et déplacer un laparoscope lors d'une opération, ledit dispositif comprenant
une unité de fixation (14, 302) destinée à fixer le dispositif (10, 200, 300) à un bras de retenue (16),
une unité de réception (32) destiné à tenir le laparoscope (100),
un corps principal (12, 202) et
un levier (30) permettant de fixer l'unité de réception (32) au corps principal (12, 202),
le corps principal (12, 202) étant monté sur l'unité de fixation (14, 302) de manière à pouvoir tourner sur un premier axe de rotation par le biais d'une première unité de rotation (26),
le levier (30) étant fixé au corps principal (12, 202) de manière à pouvoir tourner sur un deuxième axe de rotation, parallèle au premier axe de rotation, par le biais d'une deuxième unité de rotation (28), et
une première unité d'entraînement (34) étant prévue qui, lorsqu'elle est activée, entraîne les première et deuxièmes unités de rotation (26, 28) de telle sorte que le corps principal (12, 202) tourne sur le premier axe de rotation et que le levier (30) tourne sur le deuxième axe de rotation du même angle de rotation dans des sens de rotation opposés, **caractérisé en ce que**
le corps principal (12, 202) comprend un boîtier (40) qui comporte une première partie de boîtier (20) et une deuxième partie de boîtier (22) séparée de ladite première partie de boîtier (20), et
la première unité de rotation (26) est montée sur la première partie de boîtier (20) et la deuxième unité de rotation (28) est montée sur la deuxième partie de boîtier (22), et
une deuxième unité d'entraînement (24) est prévue qui permet de déplacer linéairement la deuxième partie de boîtier (22) sur une distance prédéterminée par rapport à la première partie de boîtier (20).

2. Dispositif (10, 200, 300) selon la revendication 1, **caractérisé en ce que**
la première unité de rotation (26) comprend un premier arbre (44) sur lequel l'unité de fixation (14, 302) est montée solidairement en rotation,
la deuxième unité de rotation (28) comprend un deuxième arbre (46) sur lequel le levier (30) est monté solidairement en rotation, et
les premier et deuxième arbres (44, 46) sont entraînés en rotation dans des sens opposés, du même angle de rotation, par la première unité d'entraînement (34).

3. Dispositif (10, 200, 300) selon la revendication 1 ou 2, **caractérisé en ce que** la première unité d'entraînement (34) comprend exactement un moteur (64) permettant d'entraîner les première et deuxième unités de rotation (26, 28), de préférence la première unité d'entraînement (34) comprenant un arbre principal (68), en particulier un arbre principal télescopique (68), qui est entraîné par le moteur (64) et de préférence l'arbre principal (68) entraînant les deux unités de rotation (26, 28).

4. Dispositif (10, 200, 300) selon la revendication 3, **caractérisé en ce que**
l'arbre principal (68) est en engagement avec le premier arbre (44) de la première unité de rotation (26) par le biais d'un premier ensemble formant roue tangente (74, 76) ou d'un ensemble formant pignon conique,
l'arbre principal (68) est en engagement avec le deuxième arbre (46) de la deuxième unité de rotation (28) par le biais d'un deuxième ensemble formant roue tangente (78, 80) ou d'un ensemble formant pignon conique.

5. Dispositif (10, 200, 300) selon la revendication 3, **caractérisé en ce que** le moteur (64) entraîne les unités de rotation (26, 28) par le biais d'entraînements à chaîne et/ou à courroie et/ou d'accouplement hydrauliques.

6. Dispositif (10, 200, 300) selon la revendication 1 ou 2, **caractérisé en ce que**
la première unité d'entraînement (34) comprend deux moteurs, un moteur servant à l'entraînement d'une unité de rotation (26, 28), et
une unité de commande est prévue qui commande les deux moteurs de manière synchrone.

7. Dispositif (10, 200, 300) selon l'une des revendications précédentes, **caractérisé en ce que** l'unité de réception (32) est conçue de telle sorte que le laparoscope (100) soit monté au-dessus de celle-ci sur le levier (30) de manière à pouvoir tourner librement.

8. Dispositif (10, 200, 300) selon la revendication 1, **caractérisé en ce que**
la deuxième unité d'entraînement (24) comprend un moteur (60) et un écrou de broche (63) entraîné par celui-ci, et
les première et deuxième parties de boîtier (20, 22) sont reliées l'une à l'autre par une broche (62) guidée dans l'écrou de broche (63).

9. Dispositif (10, 200, 300) selon la revendication 1 ou 8, **caractérisé en ce que** les première et deuxième parties de boîtier (20, 22) sont reliées l'une à l'autre par le biais d'au moins un guide linéaire (48, 50).

10. Dispositif (10, 200, 300) selon la revendication 1, **caractérisé en ce que** le boîtier (40) comprend un soufflet pliable (42) et/ou un capot en tôle télescopique, lesquels sont disposés entre les premier et deuxièmes parties de boîtier (20, 22).

11. Dispositif (10, 200, 300) selon la revendication 1, **caractérisé en ce que** la première partie de boîtier (20) peut coulisser au moins partiellement sur la deuxième partie de boîtier (22) ou la deuxième partie de boîtier (22) peut coulisser au moins partiellement sur la première partie de boîtier (20).

12. Dispositif (10, 200, 300) selon la revendication 1, **caractérisé en ce que** la première unité d'entraînement (34), la deuxième unité d'entraînement (24), la première unité de rotation (26) et/ou la deuxième unité de rotation (28) sont disposées chacune au moins partiellement, de préférence entièrement, à l'intérieur du boîtier (40).

13. Dispositif (10, 200, 300) selon l'une des revendications précédentes, **caractérisé en ce que**
l'unité de fixation (14, 302) comprend une première partie (82) et une deuxième partie (84), et
la première partie (82) peut tourner sur un troisième axe de rotation (18) par rapport à la deuxième partie (84), de préférence une troisième unité d'entraînement (90) étant prévue pour faire tourner la première partie (82) par rapport à la deuxième partie (84), de préférence la troisième unité d'entraînement (90) étant disposée au moins partiellement, de préférence entièrement, dans le boîtier (40) du corps principal (12, 202) ou en variante la troisième unité d'entraînement (90) étant disposée au moins partiellement, de préférence entièrement, dans un boîtier (86) de l'unité de fixation (14, 302).

14. Dispositif (10, 200, 300) selon la revendication 13, **caractérisé en ce que** le troisième axe de rotation (18) est disposé orthogonalement aux premier et deuxième axes de rotation.

15. Dispositif (10, 200, 300) selon l'une des revendications 13 à 14, **caractérisé en ce que**
le dispositif (10, 200, 300) comporte un bras de retenue (16) destiné à fixer le dispositif (10, 200, 300) à une table d'opération et
la troisième unité d'entraînement (90) est disposée à l'intérieur dudit bras de retenue (16).
